# EUROPEAN PATENT APPLICATION

(11) **EP 3 417 834 A1**
(43) Date of publication of application: **26.12.2018**
(21) Application number: 18275083.6
(22) Date of filing: 15.06.2018
(51) Int. Cl.: A61F 2/954, A61F 2/958, A61M 25/10

(54) **BALLOON AND MESH FOR LUMEN SUPPORT OR DILATION**

(30) Priority: 23.06.2017 US 201715631788
(71) Applicant: Cook Medical Technologies LLC, Bloomington, IN 47404 (US)
(72) Inventor: Eaton, Elizabeth A., Bloomington, IN 47401 (US)
(74) Representative: Williams Powell

(57) **Abstract**

A system (10) for providing lumen support and dilation includes a balloon catheter (12) having an inner balloon and an outer mesh (59) bonded to the shaft, with the mesh surrounding the inner balloon and defining an intermediate space therebetween. The mesh may have holes (34) formed in an outer wall, with the inner balloon being free from holes. The shaft (16) includes a delivery lumen in communication with the intermediate space. Daughter balloons (50) are deliverable into the intermediate space through the delivery lumen and into engagement with holes formed in the mesh, where the daughter balloons are inflated and retained by the holes of the mesh. The catheter may include one or more preloaded wires (40) extending through the delivery lumen and into the intermediate space and through the holes, such that the daughter balloons (50) are deliverable over the wires into the corresponding hole (34).

## Description

### TECHNICAL FIELD

This invention relates to endoluminal medical devices for introduction into the human or animal body for treatment of endovascular disease.

### BACKGROUND OF THE INVENTION

The functional vessels of human and animal bodies, such as blood vessels and ducts, occasionally weaken or even rupture. For example, the aortic wall can weaken, resulting in an aneurysm, or it may develop a tear in one of the layers of the aortic wall resulting in an aortic dissection.

One common surgical intervention for weakened, aneurysmal or ruptured passageways or ducts involves the introduction of a compliant balloon into the damaged blood vessel.

One type of surgical intervention utilizing the insertion of a balloon into the patient's vasculature is Percutaneous Transluminal Angioplasty (PTA), often referred to simply as angioplasty, for opening up a blocked blood vessel. This procedures involves the insertion of a balloon catheter through the vasculature and to the desired location of the blockage. The balloon is inflated and deflated at the location of the blockage, thereby opening up the blood vessel.

Another type of surgical intervention involving balloons is a procedure where a balloon catheter is introduced toward a blood vessel, such as the aorta, to repair a dissection that has occurred. In this procedure, a compliant balloon is introduced to a location adjacent the tear in the vessel wall, and the balloon is inflated to block blood flow through the "true" lumen of the blood vessel, allowing the filling/thrombosis of the "false" lumen.

In many cases, however, the damaged or defected portion of the vasculature may include a branch vessel branching from the main vessel or may be near one of these branch vessels. For example, in the case of the abdominal aorta, there are at least three major branch vessels, including the celiac, mesenteric, and renal arteries, as well as other vessels, leading to various other body organs.

Thus, in the case of a vessel blockage, it can be difficult to open up the blockage near the branch vessel or in the branch vessel itself with a traditional balloon, and it may be undesirable to inflate a balloon across an opening of a branch vessel to repair a blockage in a main vessel. In the case of a vessel dissection, inflating the balloon across a branch vessel opening may not effectively block the true lumen.

### SUMMARY OF THE INVENTION

The present invention seeks to provide an improved medical system and method.

According to an aspect of the present invention, there is provided a medical system as specified in claim 1.

More specifically, a medical system includes a balloon catheter having a shaft and a first inflatable balloon bonded to the shaft and a mesh bonded to the shaft and extending over and surrounding the first balloon. A first inflation lumen extends within the shaft and is in fluid communication with an interior cavity of the first balloon. An intermediate space is defined between an outer surface of the first balloon and an inner surface of the mesh. At least one delivery lumen extends longitudinally through the shaft and is in fluid communication with the intermediate space. The delivery lumen is configured to deliver medical devices through the shaft and into the intermediate space. The system includes at least one daughter balloon that is moveable through the delivery lumen and is configured to extend through the mesh. The mesh retains the daughter balloon when the daughter balloon extends through the mesh and is inflated. Inflation of the first balloon expands the mesh.

The mesh may include at least one hole defined in the side of the mesh. The hole is sized and configured to receive and retain the daughter when inflated. The system may also include an outer shaft that surrounds the shaft to which the balloon is bonded. The mesh may be bonded at one end to the first shaft and at its opposite end to the outer shaft. The outer shaft and the first shaft may define an annular space between then for delivering inflation fluid into the intermediate space.

The system may further include at least one wire extending through the delivery lumen and into the intermediate space. The daughter balloon may include a wire lumen and is deliverable over the wire into the intermediate space. The wire and daughter balloon are deliverable through a side of the mesh.

The system may also include multiple delivery lumens rather than a single delivery lumen. Multiple daughter balloons may be delivered through the multiple delivery lumens into the intermediate space.

The first balloon may be a compliant balloon, and the daughter balloon may be a minimally compliant balloon. The first balloon may conform to the shape of the daughter balloon when inflated.

The first balloon and mesh may be housed within a delivery sheath in a delivery configuration, and the balloon and mesh are exposed from the delivery sheath in a deployed configuration. In the delivery configuration, a wire or wires may extend into the intermediate space. The wire is moveable through a side of the mesh in the deployed configuration. The wire may extend through a side of the mesh in the delivery configuration. A terminal end of the wire may be attached to the shaft outside of the mesh in the delivery configuration.

The mesh may be coated, or the mesh may be integrally formed with a second compliant balloon. In another embodiment, a medical system may include a balloon catheter having a shaft and a first inflatable balloon bonded to the shaft and a mesh bonded to the shaft and extending over and surrounding the first balloon. A first inflation lumen extends within the shaft and is in fluid communication with an interior cavity of the first balloon. An intermediate space is defined between an outer surface of the first balloon and an inner surface of the mesh. At least one delivery lumen extends longitudinally through the shaft and is in fluid communication with the intermediate space. The delivery lumen is configured to deliver medical devices through the shaft and into the intermediate space.

The system may further include at least one wire extending through the at least one delivery lumen and into the intermediate space. The system further includes at least one daughter balloon that is moveable through the delivery lumen and is configured to extend through the mesh.

The system advantageously includes a delivery configuration with the first balloon and the mesh in a compressed delivery state and housed within a delivery sheath, and the system further includes a deployed configuration with the first balloon and the mesh exposed from the delivery sheath. In the deployed configuration, the daughter balloon is deliverable over the at least one wire and extends through the mesh and is retained in position by the mesh when the daughter balloon is inflated. Further, in the deployed configuration, inflation of the first balloon expands the mesh outward.

The wire may terminate within the intermediate space in the delivery configuration. The wire may be moveable through a side of the mesh in the deployed configuration. The wire may extend through the side of the mesh in the delivery configuration. A terminal end of the wire may be attached to the shaft outside of the mesh at an insertion end of the shaft in the delivery configuration. The above described mesh may be coated. The above described mesh may be integrated with a second compliant balloon.

According to another aspect of the present invention, there is provided a medical system comprising a balloon catheter including a shaft and a first inflatable balloon bonded to the shaft and a mesh bonded to the shaft and extending over and surrounding the first balloon; a first inflation lumen extending within the shaft and in fluid communication with an interior cavity of the first balloon; an intermediate space defined between an outer surface of the first balloon and an inner surface of the mesh; at least one delivery lumen extending longitudinally through the shaft and in fluid communication with the intermediate space, the delivery lumen configured to deliver medical devices through the shaft and into the intermediate space; at least one daughter balloon moveable through the at least one delivery lumen and configured to extend through the mesh; and at least one wire extending through the at least one delivery lumen and into the intermediate space; wherein the system includes a delivery configuration with the first balloon and the mesh in a compressed delivery state and housed within a delivery sheath, and the system further includes a deployed configuration with the first balloon and the mesh exposed from the delivery sheath; wherein, in the deployed configuration, the daughter balloon is deliverable over the at least one wire and extends through the mesh and is retained in position by the mesh when the daughter balloon is inflated; and wherein, in the deployed configuration, inflation of the first balloon expands the mesh outward.

There is also disclosed herein a method for supporting or dilating a body vessel includes delivering, to a body vessel, a balloon catheter having an inner balloon and an outer mesh each bonded to a shaft. The mesh surrounds the inner balloon and defines an intermediate space therebetween. The shaft includes an inflation lumen in communication with the inner balloon and at least one delivery lumen in communication with the intermediate space.

The method further includes delivering at least one daughter balloon through the delivery lumen into the intermediate space and into engagement with a hole formed in the mesh. The daughter balloon is inflated against the hole in the mesh. The method further includes inflating the inner balloon against the mesh and reducing the intermediate space, and expanding the mesh into engagement with the vessel wall.

The balloon catheter may include at least one wire extending through the delivery lumen and into the intermediate space. The method may further comprise routing the wire to a desired location outside of the mesh. The wire extends through the hole in the mesh after being routed to its desired location. The daughter balloon may include a wire lumen, with the daughter balloon delivered over the wire into engagement with the hole through which the wire extends.

The mesh may be bonded at one end to the shaft and at its opposite end to an outer shaft that surrounds the shaft. The intermediate space is in communication with a further inflation lumen defined between the shaft and the outer shaft. The method may further include partially inflating the mesh and increasing the intermediate space. The daughter balloons may be inflated prior to the inflating the inner balloon, and the mesh expands in response to inflation of the inner balloon.

The foregoing paragraphs have been provided by way of general introduction, and are not intended to limit the scope of the following claims. The presently preferred embodiments, together with further advantages, will be best understood by reference to the following detailed description taken in conjunction with the accompanying drawings.

### BRIEF DESCRIPTION OF THE DRAWINGS

Embodiments of the present invention are described below, by way of example only, with reference to the accompanying drawings, in which:
Figure 1 shows a balloon catheter having an inflatable balloon having holes formed in an outer wall, and a shaft having a delivery lumen in communication with the interior of the balloon, and wires extending out of the delivery lumen and through the interior of the balloon and out of the holes;
Figure 2A is a cross-sectional view of one embodiment of the shaft, showing a delivery lumen, an inflation lumen for the balloon, and a wire lumen for delivering the balloon catheter over a guidewire;
Figure 2B is another embodiment, where the shaft includes a delivery lumen and a guidewire lumen;
Figure 2C is a schematic cross-sectional view illustrating a tube that extends from the insertion end of the shaft that is integral with the shaft, with the guidewire lumen extending through the tube;
Figure 3 is a schematic view of a daughter balloon having a shaft and an inflation lumen;
Figure 4 is a cross-sectional view of the shaft of the daughter balloon;
Figure 5 is a schematic view of the daughter balloon delivered over the wire through the delivery lumen and into the balloon and out of the hole in the balloon, and inflated into engagement with the hole;
Figure 5A illustrates a reinforcing band extending around the hole in the balloon;
Figure 5B illustrates a mesh material embedded in the balloon wall around the hole in the balloon;
Figure 6 is a view of multiple daughter balloons delivered into the holes of the balloon, and the balloon inflated after each of the daughter balloons have been delivered and inflated;
Figure 7 is a cross-sectional view of the balloon catheter and the balloon in a compressed state within a delivery sheath, with wires preloaded in a delivery state;
Figure 8 illustrates the balloon catheter delivered to a body vessel and exposed from the delivery sheath, with the wires being routed into adjacent branch vessels from the holes in the balloon;
Figure 9 illustrates the daughter balloons delivered across the holes and inflated into engagement with the holes and extending into the branch vessels;
Figure 10 illustrates the balloon in an inflated condition and expanded into contact with the body vessel wall with the daughter balloons inflated into engagement with the branch vessels;
Figure 11 is a schematic view illustrating a balloon catheter having an inner balloon and a mesh surrounding the inner balloon, a shaft having a delivery lumen in communication with an intermediate space between the mesh and the inner balloon, wires extending through the delivery lumen and through the wall of the mesh, and an outer shaft surrounding the shaft;
Figure 12 is a cross-sectional representation of the balloon catheter illustrating an inflation lumen for the inner balloon and a wire lumen for delivering the balloon catheter;
Figure 13 is a cross-sectional representation of the shaft having the inflation lumen and wire lumen and four delivery lumens;
Figure 14 is a schematic illustration of the wires extending through holes in the side of the mesh and detachably retained on the end of the balloon catheter;
Figure 15 is a schematic illustration of the wires extending into the intermediate space between the mesh and the inner balloon and terminating within the intermediate space;
Figure 15A illustrates the wires piercing the mesh after being extended through the mesh;
Figure 16 is cross-sectional representation of the daughter balloons delivered into engagement with the holes in the mesh and the inner balloon in a deflated state;
Figure 17 is a cross-sectional representation of the daughter balloon having a shaft with a dual inflation lumen and a wire lumen;
Figure 18 is an illustration of the balloon of Figure 1 having mesh-reinforced areas replacing the holes in the balloon;
Figure 18A illustrates a wire after piercing the mesh reinforced area;
Figure 19 is an illustration of the balloon catheter of Figure 11 having a mesh balloon replacing the mesh, with the wires having pierced the mesh balloon;
Figure 19A illustrates the mesh balloon with pre-defined holes;
Figure 20 illustrates the balloon catheter in deployed state, with the wires routed into adjacent branch vessels and the daughter balloons delivered to their desired locations, with the inner balloon and mesh in a deflated state; and
Figure 21 illustrates the inner balloon in an inflated state, and the inner balloon and mesh expanded, with the mesh engaging the vessel wall in response to inflation of the inner balloon.

### DETAILED DESCRIPTION

Unless defined otherwise, all technical and scientific terms used herein have the same meaning as commonly understood to one of ordinary skill in the art to which this invention belongs.

The term "distal" means a location or direction that is, or a portion of a device that when implanted is further downstream in the direction of or with respect to blood flow. In the case of aortic intervention, distal means a location further away from the heart. In a trans-femoral approach, the distal end of a device is the end that is closer to the operator.

The term "proximal" means a location or direction that is, or a portion of a device that when implanted is further upstream in the direction of or with respect to blood flow. In the case of aortic intervention, proximal means a location closer to the heart. In a trans-femoral approach, the proximal end of a device is the insertion end of the device.

The term "fenestration" means an opening provided through a surface of a prosthesis from the interior of the prosthesis to the exterior of the prostheses and may have a variety of geometries, including circular, semi-circular, oval, oblong, as well as other geometries.

The term "biocompatible" refers to a material that is substantially non-toxic in the in vivo environment of its intended use, and that is not substantially rejected by the patient's physiological system (i.e., is non-antigenic). Examples of biocompatible materials from which textile graft material can be formed include, without limitation, polyesters, such as polyethylene terephthalate; fluorinated polymers, such as polytetrafluoroethylene (PTFE) and fibers of expanded PTFE, and polyurethanes. In addition, materials that are not inherently biocompatible may be subjected to surface modifications in order to render the materials biocompatible. Examples of surface modifications include graft polymerization of biocompatible polymers on the materials surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Fibers suitable for making textile grafts include polyethylene, polypropylene, polyaramids, polyacrylonitrile, nylon, and cellulose, in addition to the polyesters, fluorinated polymers, and polyurethanes as listed above. Furthermore, bioremodelable materials may also be used singly or in combination with the aforementioned polymer materials. The textile may be made of one or more polymers that do not require treatment or modification to be biocompatible. The graft may be constructed from woven multifilament polyester, for example and without limitation, Dacron™, produced by DuPONT. Dacron™ is known to be sufficiently biologically inert, non-biodegradable, and durable to permit safe insertion inside the human body.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "tubular" refers to the general shape of an endoluminal device which allows the module to carry fluid along a distance or fit within a tubular structure such as an artery. Tubular prosthetic devices include single, branched, and bifurcated devices. Tubular may refer to any shape including, but not limited to, tapered, cylindrical, curvilinear, or any combination thereof. A tubular device may have a cross-sectional shape that is, circular, substantially circular or the like. However, it should be understood that the cross-sectional shape is not limited thereto, and other shapes, such as, for example, hexagonal, pentagonal, octagonal, or the like are contemplated. The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "branch vessel" refers to a vessel that branches off from a main vessel. Examples are the celiac and renal arteries which are branch vessels to the aorta (i.e., the main vessel in this context). As another example, the hypogastric artery is a branch vessel to the common iliac, which is a main vessel in this context. Thus, it should be seen that "branch vessel" and "main vessel" are relative terms.

"Longitudinally" refers to a direction, position or length substantially parallel with a longitudinal axis of a reference, and is the length-wise component of the helical orientation.

"Circumferentially" refers to a direction, position, or length that encircles a longitudinal axis of reference. The term "circumferential" is not restricted to a full 360° circumferential turn or to a constant radius.

The terms "patient," "subject," and "recipient" as used in this application refer to any animal, especially humans.

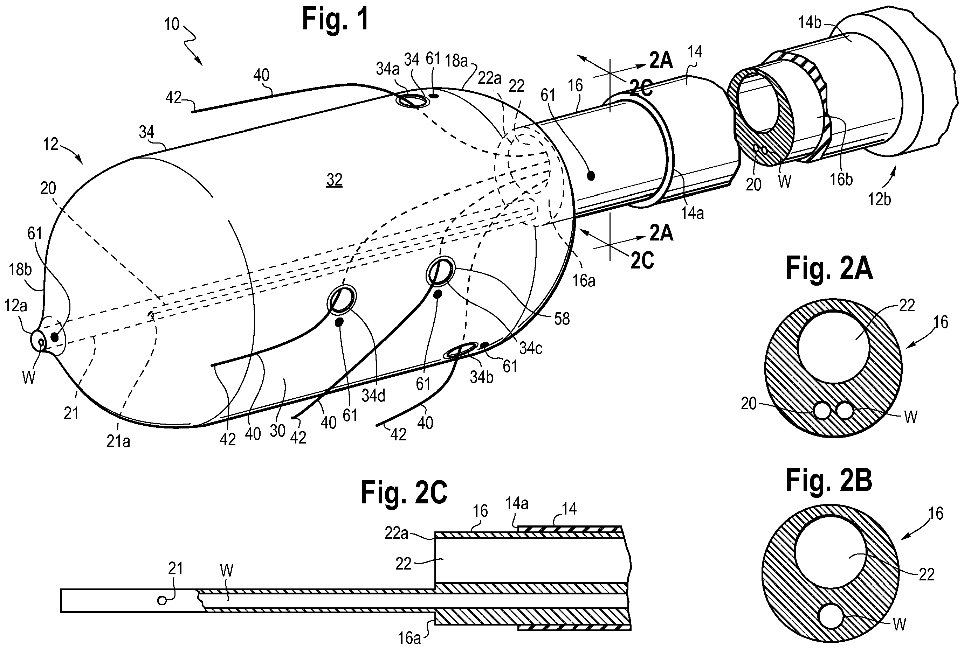

Figures 1-10 show a system 10 including a balloon catheter 12 and a delivery sheath 14 for delivering and deploying the balloon catheter 12 within a patient's vasculature at a desired location. As used herein, references to an insertion end refer to the end of a device or component that is inserted first into the patient and that is opposite an operator end, which is the end that typically remains out of the body.

Figure 1 is a schematic illustration of a balloon 18 in an expanded or partially expanded state that includes holes 34 allowing for additional balloons to pass through at least partially through a wall 30 of the balloon 18. These additional balloons are not shown in Figure 1 in order to illustrate the holes 34 and other structure. Figure 1 therefore illustrates the balloon 18 in an expanded condition, but in use, the balloon 18 typically remains in a compressed position prior to the additional balloons being delivered through the holes 34 to seal the holes 34. However, in some approaches, the balloon 18 may be expanded into a partially expanded state to aid in the introduction of the further balloons, as further described below.

The delivery sheath 14 includes an insertion end 14a and an operator end 14b. The balloon catheter 12 likewise includes an insertion end 12a and an operator end 12b.

The balloon catheter 12 includes a main tubular shaft 16 that extends longitudinally between an insertion end 16a and an operator end 16b, and an inflatable balloon 18 bonded to the shaft 16 near the insertion end 16a of the shaft 16. As shown in Figure 1, the insertion end 12a of the overall balloon catheter 12 is disposed further away from the operator than the insertion end 16a of the shaft 16.

The shaft 16 defines an inflation lumen 20 that is in fluid communication with an interior cavity of the balloon 18. In one approach, the inflation lumen 20 may be defined by the shaft 16 and a tube 21 that extends from the insertion end 16a of the shaft 16 and into the interior of the balloon 18 and to the insertion end 12a of the balloon catheter 12. The shaft 16 and the tube 21 may define an inflation port 21 a that delivers inflation fluid from the inflation lumen 20 into the interior of the balloon 18, as shown in Figure 1. In one approach, the tube 21 may be a separate component that attaches to the end of the shaft 16, as illustrated in Figure 1. Alternatively, as shown in Figure 2C, the tube 21 may be an integral extension of the shaft 16. In one approach, as shown in Figure 2C, the tube 21 may provide support for the balloon catheter 12, and may include the guidewire lumen W, but where inflation fluid is provided through another lumen such as a delivery lumen 22, as described below.

The shaft 16 further defines a delivery lumen 22 extending longitudinally through the shaft 16 and configured to allow other system components to be housed therein or delivered therethrough. The catheter 12 and shaft 16 may be delivered with or without the use of a guidewire. In one another approach, the balloon catheter 12 may include a guidewire lumen W formed in the shaft 16 and extending through the tube 21 or other similar support structure that extends through the balloon 18, as shown in Figures 1-3. It will be appreciated that the various lumens can be arranged in a variety of ways in the shaft 16 and the through the balloon 18, such that the delivery lumen 22 opens into the interior of the balloon 18 and the inflation lumen 20 can provide inflation fluid to the interior of the balloon, with the guidewire lumen W extending through the balloon 18 and isolated from the inflation lumen 20. One example of a lumen arrangement is shown in Figure 2A, which shows a cross-section of the shaft 16 and the lumens extending therethrough, including guidewire lumen W, delivery lumen 22 and inflation lumen 20.

Figure 2B illustrates an embodiment where the shaft 16 includes the delivery lumen 22 and a guidewire lumen W, but without a separate inflation lumen. In this embodiment, inflation fluid may be delivered through the delivery lumen 22. The guidewire lumen W extends through the tube 21 in this embodiment, as shown in Figure 2C.

In one embodiment, the delivery lumen 22 is sized to be larger/wider than the inflation lumen 20. In particular, the delivery lumen 22 is sized to be wide enough to facilitate delivery of additional balloons through the delivery lumen 22, as well as multiple guidewires for each of the additional balloons, as further described below.

The balloon 18, attached to the insertion end 12a of the catheter 12 as well as to the insertion end 16a of the shaft 16, is preferably in the form of a compliant balloon, meaning that the balloon 18 will typically take the shape of the vessel in which it is deployed once inflated. The balloon 18 is preferably sized to correspond generally to the size of the vessel to which the balloon 18 will be delivered and inflated. The balloon 18 being in the form of a compliant balloon allows the balloon 18 to be inflated to occlude or fill a target blood vessel while limiting instances where the balloon 18 may cause further damage to the vessel wall when inflated. The compliant balloon 18, when inflated, will tend to take the shape of the blood vessel due to its compliant structure. The compliant, or semi-compliant in another approach, balloon 18 helps the balloon 18 accommodate variation in the vascular anatomy that may vary from patient to patient.

In the case of a traditional inflatable balloon, the balloon wall is typically intact such that the balloon will retain the inflation fluid that is introduced into the cavity defined by the balloon to inflate the balloon.

As shown in Figure 1, the balloon 18 includes the wall 30 that defines an interior cavity 32 therein. The interior cavity 32 is in fluid communication with the inflation lumen 20 of the catheter 12 and the delivery lumen 22 of the shaft 16, such that inflation fluid can be introduced into the interior cavity 32 via the inflation lumen 20 or the delivery lumen 22 to inflate and expand the balloon wall 30 in a manner known in the art, as well as allowing further medical devices to be introduced into the interior cavity 32 via the delivery lumen 22.

Furthermore, the balloon 18 defines one or more holes 34, or punctures or passageways or the like, in the wall 30 that permit the passage of additional structure through the wall 30. Accordingly, with the holes 34 extending through the wall 30, the balloon 18 differs from a traditional balloon in that inflation fluid introduced in a balloon with holes would leak out of the balloon absent other structure that will seal the holes. In the present approach, such structure is provided in the form of additional balloons, which are further described below. In one approach, the balloon wall 30 may include a reinforcing band 58, further described below, that surrounds each of the holes 34 to provide reinforcement to the holes 34 in response to additional balloons being inserted through the holes 34.

In one approach, the holes 34 are generally small. Exemplary holes may be about 2-4 mm in width. The size of the holes 34 are preferably selected to be smaller than the size of the ultimate structure that will be extended through the hole 34, such that after the structure is extended through the hole 34 and left in place, the holes 34 will be generally sealed due to the larger size of the inserted structure exerting a radially outward force on the holes 34, such that the interior cavity 32 within the balloon 18 may still be inflated in response to the introduction of inflation fluid. Accordingly, in one approach, 2-4 mm sized holes are one preferred sizing to accommodate a further balloon that inflates to about 8 mm in width, for example.

In one approach, the balloon 18 may include four holes 34a, 34b, 34c, and 34d. The holes 34a-d are located on the balloon 18 such that their location will typically correspond to the general location of branch vessels in the target delivery and deployment area. For example, the four-hole arrangement may be used in the abdominal aorta near the left and right renal arteries (LRA and RRA) and the supermesenteric artery (SMA) and celiac artery (CA). The SMA and CA are typically disposed above a patient's renal arteries, such that they are between a patient's renal arteries and the heart.

Thus, for a balloon 18 that is designed and arranged to be delivered to this area of the patient, holes 34a and 34b can be arranged on laterally opposite sides of the balloon 18 to accommodate the LRA and RRA, with holes 34c and 34d being disposed longitudinally offset from the holes 34a and 34b and generally on the same lateral side of the balloon 18 as each other. Differing anatomy may result in altering the arrangement of the holes 34 as needed. Further, depending on the desired location for introduction and inflation of the balloon 18, additional holes or fewer holes may be used.

The balloon 18 defines a first end 18a and a second end 18b. The first end 18a is preferably attached to the insertion end 16a of the shaft 16, with the second end 18b being bonded to the insertion end 12a of the balloon catheter 12 at the opposite longitudinal end of the balloon 18 opposite the interface between the insertion end 16a of the shaft 16 and the first end 18a of the balloon 18. The delivery lumen 22 includes an opening 22a at the insertion end 16a of the shaft 16 that is in fluid communication with the interior cavity 32 of the balloon, such that wires or other structure can be passed through the delivery lumen 22 and into the interior cavity 32. The delivery lumen 22 therefore could be used for providing inflation fluid as an alternative to the inflation lumen 20 and tube 21, with the tube 21 being used for support rather than inflation, as described above, and the tube 21 may include the guidewire lumen W but remain fluidly isolated from the cavity 32 of the balloon 18, as shown in Figure 1B.

As shown in Figure 1, the system 10 may further include one or more wires 40 for assisting in the delivery of additional structure to the holes 34. The wires 40 will act as guidewires for the additional structure to allow for the additional structure to be routed to the desired hole 34. The number of wires 40 preferably corresponds to the number of holes 34 in the balloon 18. However, it will be appreciated that the number of wires 40 could differ from the number of holes 34 in some cases. Typically, each hole 34 will have a corresponding wire 40 extending through the hole 34.

Thus, in one approach, the wires 40 extend through the delivery lumen 22 and out of the insertion end 16a of the shaft 16 and into the interior cavity 32 defined by the balloon 18. Each individual wire 40 may further extend through a corresponding hole 34 of the balloon 18 and out of the interior cavity 32 of the balloon 18, such that a terminal end 42 of the wire 40 is disposed outside of the interior cavity 32. The wires 40 are preferably arranged in a pre-loaded state, such that they extend though the holes 34 of the balloon while the balloon 18 is housed within the delivery sheath 14 prior to insertion into the body. The wires 40 may be preloaded as packaged and provided to the doctor in a pre-loaded state, or the wires 40 may be loaded by the doctor prior to delivery of the catheter 12 into the patient. In either case, the wires 40 are preloaded in the catheter 12 in a delivery configuration prior to insertion into the patient. Thus, by being pre-loaded, the wires 40 may already extend out of the holes 34 and will not need to be routed through the generally small holes 34 of the balloon 18 after the balloon 18 is exposed from the sheath 14 and delivered to the desired delivery area. Accordingly, the wires 40 being pre-loaded will result in the wires 40 extending through the holes 34 prior to the balloon 18 being inflated.

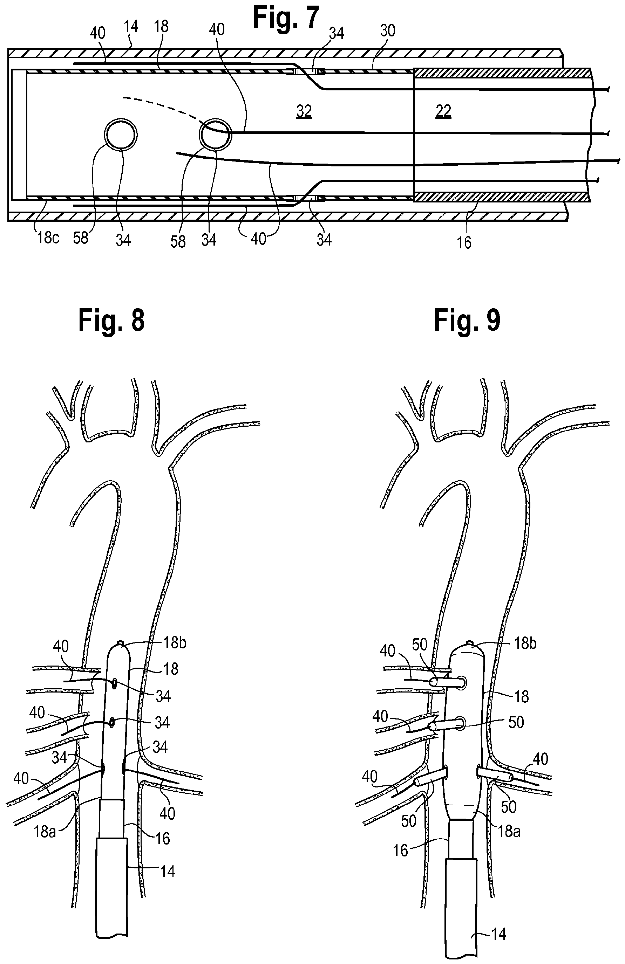

In another approach, the pre-loaded wires 40 may terminate within the balloon cavity 32 when the balloon catheter 12 is delivered in the delivery configuration, and the wires 40 may be carefully routed through the holes 34 after the balloon 18 has been exposed within the body lumen. In this approach, the balloon 18 may be partially inflated to increase the size of the cavity 32 to aid in routing the wires 40. In another approach, the wires 40 may not be pre-loaded and may be introduced through the balloon catheter 12 and into and through the cavity 32 after the balloon 18 has been delivered. Figure 7 illustrates wires 40 both extending through the holes 34 in the preloaded state and terminating within the cavity 32 in the preloaded state.

Thus, with the wires 40 extending from the delivery lumen 22 through the cavity 32 and out of the balloon 18 to the exterior of the balloon 18, additional components can be delivered along the wires 40 and will be routed to the corresponding hole 34 through which the wires 40 extend.

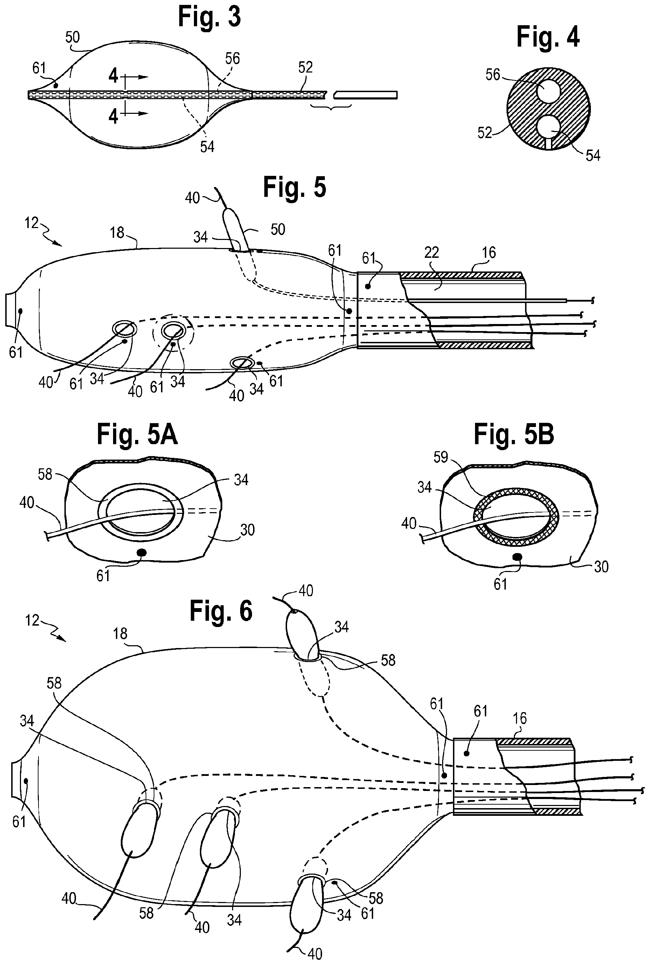

With reference now to Figures 3-6, the system 10 further includes one or more "additional," "secondary," or "daughter" balloons 50 that are configured to be delivered through the delivery lumen 22 over the wires 40 and into engagement with the holes 34 defined by the balloon 18.

Figures 3 and 4 shown examples of daughter balloons. The daughter balloons 50 may be attached to a shaft 52 having an inflation lumen 54 and a wire lumen 56 in the manner of a traditional balloon catheter. The inflation lumen 54 provides inflation fluid to the interior of the balloon 50 to inflate and expand the balloon 50 in a manner known in the art, and the balloon 50 and shaft 52 are deliverable over a wire via the wire lumen 56 in a known manner as well. Figure 4 illustrates one example of a lumen arrangement, showing the shaft 52 in cross-section. However, it will be appreciated that other arrangements of the lumens could be used, such as a dual lumen design for the inflation lumen 54, or a coaxial lumen design where the wire lumen 56 is disposed coaxially within the inflation lumen 54.

Thus, with reference to Figure 5, in one approach, individual daughter balloons 50 are deliverable over individual wires 40 through the delivery lumen 22 of the shaft 16 and into engagement with the hole 34 that corresponds to the wire 40 over which the particular individual balloon 50 was delivered. Figure 5 illustrates a first daughter balloon 50 being delivered to one of the holes 34 over the wire 40 that extends through the hole 34. Figure 5 further illustrates the other holes 34 having wires 40 extending therethrough. Figure 5 illustrates the balloon 18 in a partially inflated state, however delivery of the daughter balloons 50 may occur when the balloon 18 is still in a compressed state and prior to any inflation. It may be difficult to inflate the balloon 18 effectively prior to delivery of each of the daughter balloons 50 due to the holes 34 being present in the balloon 18.

The daughter balloons 50 may be made from traditional medical balloon materials, and can be compliant or semi-compliant, depending on the needs of the user. The size of the daughter balloons 50 can also be selected to correspond to a patient's particular anatomy. As described above with respect to the holes 34, the expanded width of the balloon 50 is preferably greater than the size of the hole 34, such that when the balloon 50 is expanded into engagement with the hole 34, the edge of the hole 34 will seal against the outer wall of the daughter balloon 50. The compliant nature of the balloon 18 will allow the hole 34 to stretch to accommodate the expanded outer width of the daughter balloon 18. In one approach, the daughter balloons 50 are more rigid than the balloon 18, such that the daughter balloons 50 will stretch the holes 34 in the balloon 18, as shown in Figure 5. In another approach, the daughter balloons 50 may be less rigid, and in this case the holes 34 may not stretch, and instead the daughter balloons 50 would expand further on each side of the hole 34 than in the hole 34, such that the diameter of the daughter balloon 50 on either side of the hole 34 is greater than the diameter of the hole 34, thus taking on a somewhat hourglass shape, as shown in Figure 6.

The balloons 50 are preferably delivered over the wires 40 in a sequential manner, such that the delivery lumen 22 of the shaft 16 can be sized to accommodate the number of preloaded wires 40 in addition to allowing a single balloon 50 to be delivered through the lumen 22, which keeps the overall width of the shaft 16 small. However, it would also be possible to increase the size of the delivery lumen 22 to allow for delivery of more than one balloon 50 at a time side-by-side, but this would also increase the width of the shaft 16 to a size larger than one where balloons 50 are delivered sequentially.

Figure 6 illustrates the balloon 18 in a fully inflated state after each of the daughter balloons 50 have been delivered to the corresponding holes 34 over the corresponding wires 40. The balloons 50 have been inflated and have created a seal with the holes 34, thereby sealing off the cavity 32 inside the balloon 18 such that the balloon 18 may be inflated. Figure 6 illustrates the example of the daughter balloons 50 taking on an hourglass shape after inflation, as described above.

Due to the expansion of the balloons 50 while extending through the holes 34 to create the seal, the balloon 34 may also include the reinforcing band 58 disposed around the edge of the holes 34, as shown in Figure 5A. The reinforcing band 58 can help prevent tearing of the balloon 18 at the location of the holes 34 when the holes 34 are stretched, and can also help provide a seal against the expanded daughter balloon 50. As shown in Figure 5A, the reinforcing band 58 can be in the form of an additional layer of balloon material that is bonded or adhered to the area surrounding the hole, or it could be in the form of an applied coating or curing adhesive. In another approach, as shown in Figure 5B, a mesh material 59 may be embedded in the wall 30 of the balloon 18 around the holes 34.

When the daughter balloons 50 are expanded into a sealing engagement with the holes 34 of the balloon 18, the balloon 18 will be generally sealed from inflation fluid leaking out of the balloon 18 when the balloon 18 is inflated. It has been found that the use of 8x20 mm sized daughter balloons 50 inserted into 2-4 mm holes 34 in the balloon 18 allows the balloon 18 to hold greater than 1 atm of pressure when inflation fluid is introduced into the cavity 32 to inflate the balloon 32. As shown in Figure 5, the balloon 18 and the holes 34 thereof, the daughter balloons 50, and shaft 16, as well as other structure described above, can each include radiopaque markers 61 disposed at various selected locations to aid in locating the balloon 18 and the various corresponding and cooperating structure at the desired location within the patient's anatomy. For example, markers 61 may be located at each of the holes 34 to assist in positioning the balloon 18 so that the wires 40 and daughter balloons 50 may be routed to the desired branch vessels. Similarly, the wires 40 may be made of a radiopaque material.

The daughter balloons 50 can be used to cannulate various branch vessels adjacent the delivered location of the main balloon 18. This can be performed quickly and easily due to the pre-loaded wires 40 that extend through the balloon 18 in its delivery state. The wires 40 are moveable relative to the shaft 16 and the holes 34 of the balloon 18, such that after the balloon 18 and wires 40 have been delivered, the wires 40 can be individually extended into the desired branch vessel prior to delivering the daughter balloon 50. Accordingly, the daughter balloon 50 will enter the desired branch vessel along the wire 40.

The system 10 has a delivery state and a deployed state. With reference to Figure 7, in the delivery state, the balloon 18 and shaft 16 and wires 40 are disposed within the delivery sheath 14 and covered by the delivery sheath 14. The wires 40 are pre-loaded in the balloon 18. Figure 7 shows three wires 40 extending through the holes 34 in the balloon 34 such that they extend through the holes 34 and to the exterior of the balloon 18 while in the delivery state. Figure 7 also illustrates one wire 40 terminating within the balloon cavity 32. It will be appreciated that all of the wires 40 may extend out of the holes 34 in the delivery state, all of the wires 40 may terminate with the cavity 32 in the delivery state, or some of the wires 40 may extend out of the holes 34 and others of the wires 40 may terminate within the balloon cavity 32.

In the deployed state, shown in Figure 8, the delivery sheath 14 is retracted relative to the balloon 18, shaft 16, and wires 40, thereby exposing the balloon 18 and the wires 14 to the surrounding vasculature. From this deployed state, the wires 40 may be routed into the desired branch vessels, and the balloons 50 may be introduced as described above and the balloon 18 may be inflated.

As shown in Figure 8, upon the balloon 18 being exposed by retracting the sheath 14 at the desired location, the wires 40 are routed into the desired adjacent branch vessels. As shown in Figure 9, following positioning of the wires 40, the daughter balloons 50 are delivered over the wires 40 and into the branch vessels while also being disposed within the holes 34 of the balloon 18. The balloons 50 are preferably delivered sequentially. However, as described above, in another approach multiple balloons 50 may be delivered side-by-side or otherwise together if the delivery lumen 22 is wide enough to accommodate multiple daughter balloons 50 in that arrangement.

After delivering the daughter balloons 50 over the wires 40 and into the holes 34 of the balloon 18, the daughter balloons 50 are inflated. The daughter balloons 50 can be inflated sequentially after delivering each individual balloon 50, or multiple balloons 50 may be inflated at the same time after delivering multiple balloons 50. In another approach, the balloons 50 could each be inflated at the same time after some or all of the balloons 50 have been delivered.

As the balloons 50 are inflated, they can be inflated to provide support to the branch vessels. In another approach, the balloons 50 may be positioned at different areas outside of the balloon 18 outside of a branch vessel. For example, one or more of the balloons 50 could be positioned against the main vessel wall.

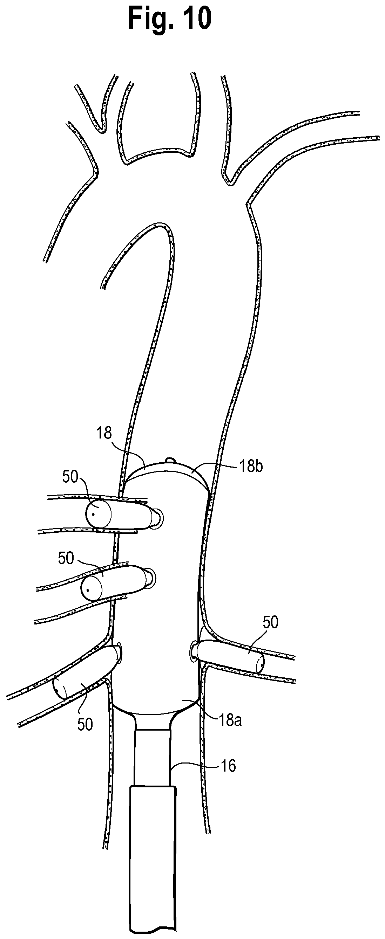

As shown in Figure 10, after each of the daughter balloons 50 have been inflated, the main balloon 18 is inflated and expanded into engagement with the main vessel wall. The previous inflation of the daughter balloons 50 provides a sealing and filling of the holes 34, such that the main balloon 18 will sufficiently inflate.

In one approach, the balloon 18 is compliant, and the daughter balloons 50 are minimally compliant, meaning that the daughter balloons 50 can inflate to a predefined shape, causing the balloon 18 to stretch in response to inflation of the daughter balloons 50. Therefore, when the balloon 18 is inflated, it will tend to conform to the shape of the vessel wall as well as around the daughter balloons 50. In another approach, the daughter balloons 18 may be compliant and will take the shape of the vessel in which they are deployed, and inflation of the balloon 18 may alter the shape of the daughter balloons 50, depending on whether the balloon 18 or daughter balloons 50 are more rigid relative to each other.

The main balloon 18 can be cycled between an inflated and deflated position to open up a blocked blood vessel. In this case, the daughter balloons 50 provide support to the branch vessels as the main balloon 18 is inflated and deflated. While the balloon 18 has been described as being compliant and conforming to the shape of the vessel, it will be appreciated that the balloon 18 may still be arranged to have sufficient rigidity when inflated to open up a blocked vessel.

In the case of aortic dissection, such as type B dissection, the daughter balloons 50 in their inflated state provide support to the branch vessels, while the inflation of the main balloon 18 provides support within the true lumen, thereby allowing for filling the false lumen with embolic material. It will be appreciated that the above arrangement may be used in various other situations where balloon support for a main vessel and branch vessels is desired.

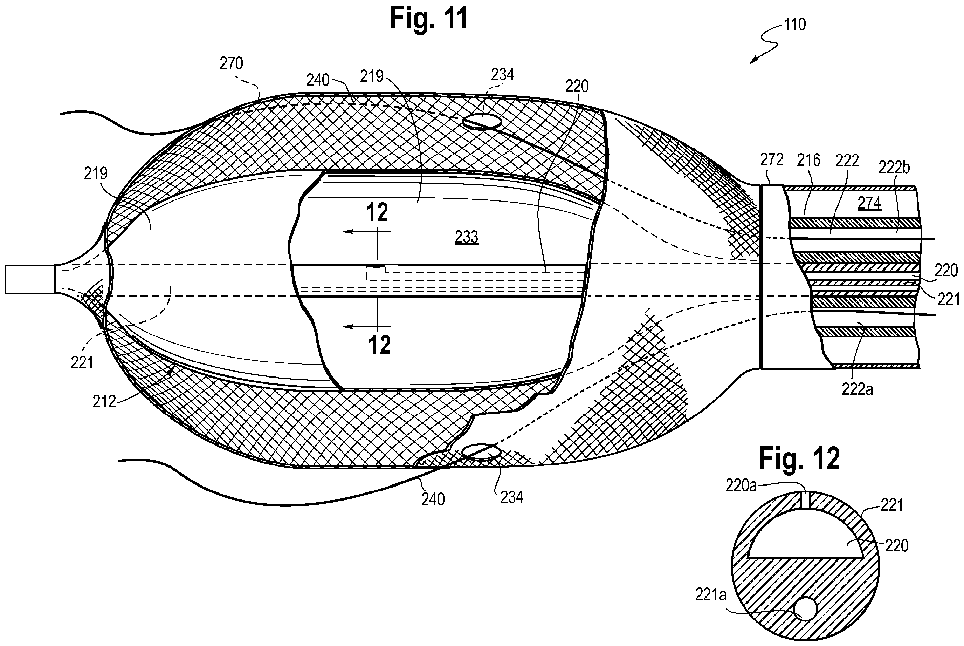

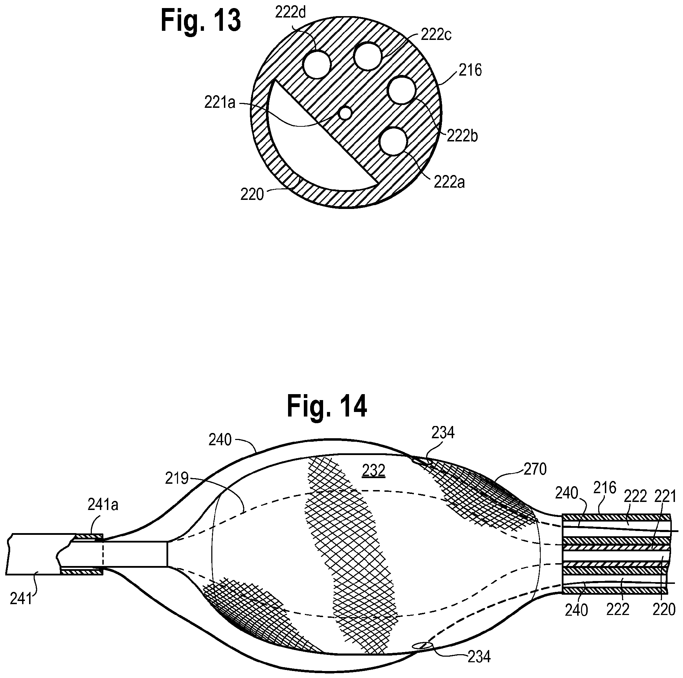

Another embodiment is shown in Figures 11-21. As shown in Figures 14, 15, 19 a system 210 can include a balloon catheter 112 having a compliant balloon 219, and can further include a mesh 270, where the mesh 270 subsumes the compliant balloon 219. Put another way, the mesh 270 is an outer support structure that will retain daughter balloons, and the balloon 219 is an inner balloon that provides inflation force to the mesh 270. Unlike the balloon 18, the balloon 219 does not include any holes or other ports for accepting a daughter balloon. Rather, the daughter balloons are installed through the mesh 270.

The mesh 270 may be formed of a woven Nickel-Titanium metal, which may be covered in a silicone, urethane, or similar coating. Alternatively, the mesh 270 could be formed of covered fibers. The mesh 270 is preferable coated and covered, but a bare mesh could also be used, if desired.

The mesh 270 and the balloon 219 define a space or cavity 232 between them. The mesh 270 may include holes 234 that allow fluid communication between the cavity 232 and the area outside the mesh 270, similar to the balloon 18 described above. However, in another approach, the mesh 270 may not include any specific holes or passageways, other than the cells that may be defined by the mesh 270. In the case of a covered mesh 270, the cells may be filled by the coating or covering.

As shown in Figures 11 and 12, the catheter 212 includes a shaft 216 having an inflation lumen 220 in fluid communication with a cavity 233 defined within the inner balloon 219. The catheter 212 may further include a tubular support 221 that extends through the cavity 233 of the inner balloon 219, where the tubular support 221 includes the inflation lumen 220 having a port 220a for delivering inflation fluid to the cavity 233. The tubular support 221 may also include a wire lumen 221 a extending therethrough, which extends fully through the catheter 112 such that the catheter 112 can be delivered over a guidewire via the wire lumen 221 a. One example of this arrangement can be seen in Figure 12. Of course, other arrangements of inflation lumens and guidewire lumens for the internal balloon 219 may also be used, such as a coaxial arrangement.

The shaft 216 includes a delivery lumen 222, similar to the lumen 22 described above. In another approach, the delivery lumen 222 could be in the form of multiple daughter balloon delivery lumens 222a, 222b, 222c, 222d for separating individual wires 240 used for delivering daughter balloons 250, as well as the wire lumen 221a for delivering the catheter 212 and shaft 216 to a desired location.

One example of the arrangement of lumens in the shaft 216 is illustrated in Figure 13, which shows the multiple delivery lumens 222a, b, c, and d, the wire lumens 221a, and the inflation lumen 220.

Due to the inflation of the inner balloon 219 to provide the main inflation function, the system 210 can be arranged without a dedicated inflation lumen in shaft 216 for the mesh 270 or the intermediate space 232 defined between the mesh 270 and the inner balloon 219..

However, some inflation of the mesh 270 may be desirable or possible in some instances. Accordingly, as shown in Figure 11, the system 210 can include an outer shaft or sheath 272 that surrounds the shaft 216, providing a co-axial inflation lumen 274 for providing inflation fluid to the interior of the mesh 270 if necessary or desired. The outer shaft 272 in this approach will support the mesh 270, rather than the mesh being supported by the shaft 216. Thus, while both ends of the balloon 219 may be bonded to the catheter 212, the mesh 270 may be bonded at one end to the insertion end of the catheter 212 and at the opposite end to the outer shaft 272.

However, in another approach, the mesh 270 can be bonded at both ends to the shaft 216 and can include no dedicated inflation lumen or inflation path for the space between the mesh 270 and the inner balloon. In this approach, inflation fluid could be applied via one of the delivery lumens 222, if necessary, prior to filling the delivery lumen with a daughter balloon 250.

The wires 240 can be pre-loaded similar to the wires 40. As shown in Figure 14, the wires 240 may be preloaded and extend through the holes 234 and out of the mesh 270.

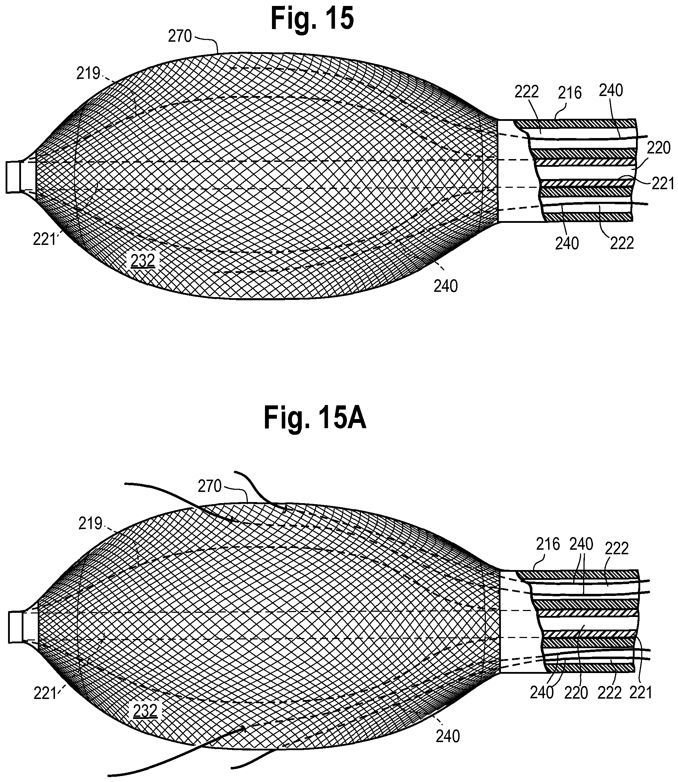

However, in another approach as shown in Figure 15, the mesh 270 can be configured without any pre-formed holes. In this approach, the wires 240 can be preloaded such that their ends terminate within the cavity 232 but do not extend outside the cavity 232. The wires 240 themselves can be used to puncture the mesh 270 to form the holes 234 after the balloon 219 and mesh 270 have been delivered to the target site. Once holes 234 are formed in the mesh 270, the general operation of delivering daughter balloons 250 to the holes 234 can be performed in a similar manner. Figure 15A illustrates the wires 240 puncturing the mesh 270.

Thus, the mesh 270 and the wires 240 can be used to accommodate patient anatomy that may differ from a traditional anatomy, or to allow for delivery of the mesh 270 and balloon 219 and the daughter balloons 250 to an atypical location. This configuration can also be used for traditional or expected anatomy arrangements, but when it may be unclear whether a branch vessel would benefit from receiving a daughter balloon. Accordingly, this configuration provides added flexibility in determining the quantity and location of the holes 234 that are defined in the mesh 270 after the mesh 270 has been delivered.

As shown in Figure 14, in the case where the wires 240 are preloaded and extend through pre-formed holes 234, the terminal ends of the wires 240 may be detachably fixed to a wire support 241 of the shaft 216 or balloon catheter 212 that extends beyond the end of the balloon 219. In this approach, the terminal ends extending outside of the mesh 270 can be retained such that they are not loose during delivery. To detach the wires 240, the wires 240 can be pulled back toward the user and then fed into the desired branch vessel or other desired location. This securement arrangement could also apply to the wires 40 described above.

The wires 240 may retained by being inserted into holes or passages of the wire support 241. They may also be detachably bonded to the wire support 241. In another approach, the terminal ends of the wires 240 may be retained by a sleeve 241a that can be moveable relative to the support 241, or the wires 240 may simply be retracted out of the sleeve 241a. In any case, to route the wires into a desired location outside of the mesh 270, the wires 240 are detachable from the support 241 and can be routed as necessary.

To deliver the daughter balloons 250, the process is similar to that described above regarding the daughter balloons 50. The daughter balloons 250 in this case are delivered through the delivery lumen 222 or lumens and into the holes 234, and are then inflated. If inflation of the mesh 270 is possible via the outer shaft 272 or via one or more delivery lumen, the mesh 270 may be partially inflated to separate the mesh 270 further from the inner balloon 219.

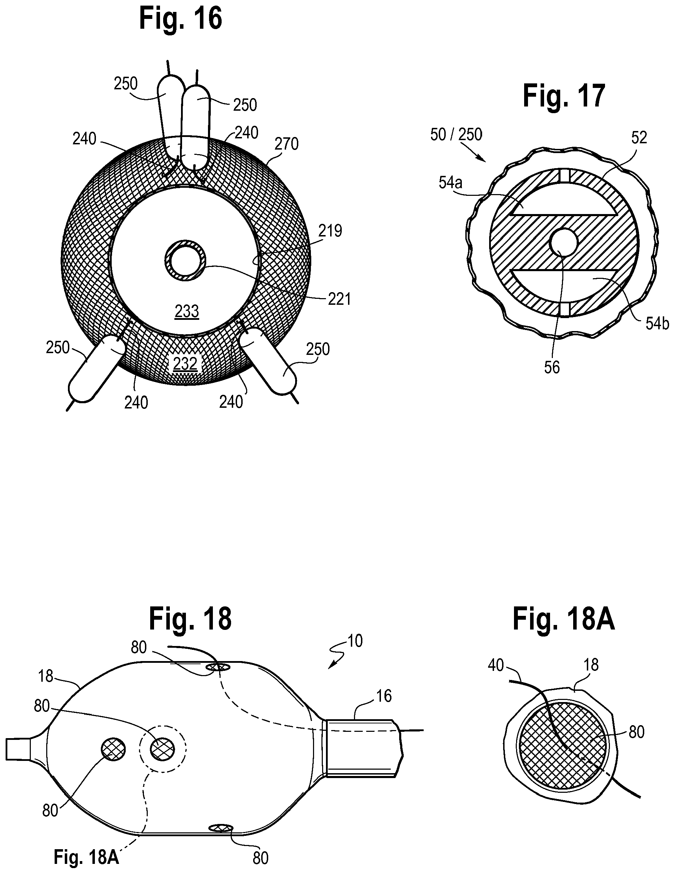

Figure 16 is a cross-sectional view that illustrates an example of the daughter balloons 250 being delivered into the holes 234 over the wires 240, which are extended through the holes 234. The daughter balloons 250 are inflated into engagement with the mesh 270. The inner balloon 219 is shown in a deflated state. The inner balloon 219 is subsequently inflated to fill the body vessel, but because the inner balloon 219 does not include the holes 234, inflation can be achieved even without a seal between the daughter balloons 250 and the holes 234.

The inner balloon 219 is the desired balloon for providing the main inflation for the system 210, while the mesh 270 provides the structural support for the daughter balloons 250. Thus, once the daughter balloons 250 have been delivered to the desired location and inflated, the inner balloon 219 is inflated to fill the blood vessel. The compliant nature of the inner balloon 119 causes the balloon 219 and the mesh 270 surrounding the balloon 219 to take the shape of the vessel wall and support the vessel wall.

As described above, the mesh 270 provides structural support to the balloons 250. Accordingly, the balloons 250 are deliverable through the shaft 216 along the wires 240 and into the cavity 232 between the mesh 270 and the inner balloon 219. The balloons 250 are further delivered such that the balloons 250 are disposed across and through the mesh 270. The balloons 250 are inflated and expanded, and their expansion provides an engagement with the mesh 270 and the holes 234 that were either predefined or defined by the wires 240 puncturing the mesh 270 and defining the holes 234.

The use of the inner balloon 219 to provide the bulk of the inflation allows for the wires 240 to be used to puncture the holes 234 in the mesh 270 to define the holes 234. Similarly, the wires 240 could also be used to create additional holes in addition to predefined holes 234 in the event it is desirable to place a daughter balloon in a different location. The existence of additional and unfilled holes 234 is not detrimental to inflation of the inner balloon 219 and mesh 270, because the inner balloon 219 provides the inflation and the mesh 270 serves as the support for the daughter balloons 250.

However, the mesh 270 could also be inflatable if desired, with inflation fluid provided by the outer shaft 272. In this case, the mesh 270 may not hold as much pressure as the inner balloon 219, but providing inflation fluid to the mesh 270 could assist with occlusion or mobility of the daughter balloons 250 or wires 240. Similarly, inflation fluid could be provided into the mesh 270 to separate the mesh from the inner balloon 219 to create a larger intermediate space between the inner balloon 219 and the mesh 270 to assist in moving the daughter balloons 250 into engagement with the mesh 270. Inflation fluid may also be introduced into the cavity 233 via the delivery lumen 222 that is used to deliver the daughter balloons 250.

The use of the combination balloon 219 and mesh 270 may be desirable in some instances and can provide added functionality relative to the single balloon system 10 described above, as the balloon and mesh combination does not include a sealing interface between the daughter balloons 250 and the mesh 270, because the inner balloon 219 is sealed.

In addition to using the daughter balloons 250 (or 50) to fill branch vessels, the daughter balloons 250 can be used to push the balloon 18 or 219 away from the vessel wall on one side, thereby providing a flow path along one side while the opposite side remains pushed against the vessel wall. To provide this sort of pushing ability, the daughter balloons 50 or 150 are preferably made from a minimally compliant material, such that they will be inflated to a predetermined shape and will be less apt to conform to the shape of the surrounding anatomy.

The daughter balloons 50 and 250 have been described as having an inflation lumen 54 and a wire lumen 56. In one approach, as illustrated in Figure 17, the inflation lumen 54 is in the form of a dual lumen 54a and 54b, such that inflation can occur even in a situation where the shaft of the daughter balloon 50 or 250 is kinked.

In another embodiment shown in Figure 18, rather than use a separate mesh 270 that surrounds the balloon 219, the system 10 having the balloon 18 may include mesh-like reinforced areas 80 on the balloon 18 without any predefined holes. In this approach, the wires 40 may puncture through the mesh-reinforced areas 80, as shown in Figure 18. Figure 18A illustrates the mesh reinforced area 80 having a hole 34a created by the wire 40 having punctured the mesh reinforced area 80.

For example, the balloon 18 may include one or more cross-hatched reinforcement areas 80 in the form or fibers or metal strands. The reinforcement areas 80 may be applied during the forming of the compliant balloon 18. The reinforcement areas 80 can be located in desired locations, and puncturing the balloon 18 to form a passageway for the daughter balloons 50 can be performed at a later time through these desired areas, as described above with reference to the system 210. Thus, the application of reinforcement areas 80 can replace the use of pre-formed holes through the balloon 18.

The cellular nature of the reinforcement areas 80 can limit the propagation of the resulting holes through the surface of the compliant balloon 18, thereby reinforcing the area of the puncture and limiting tearing, over-stretching, or the like.

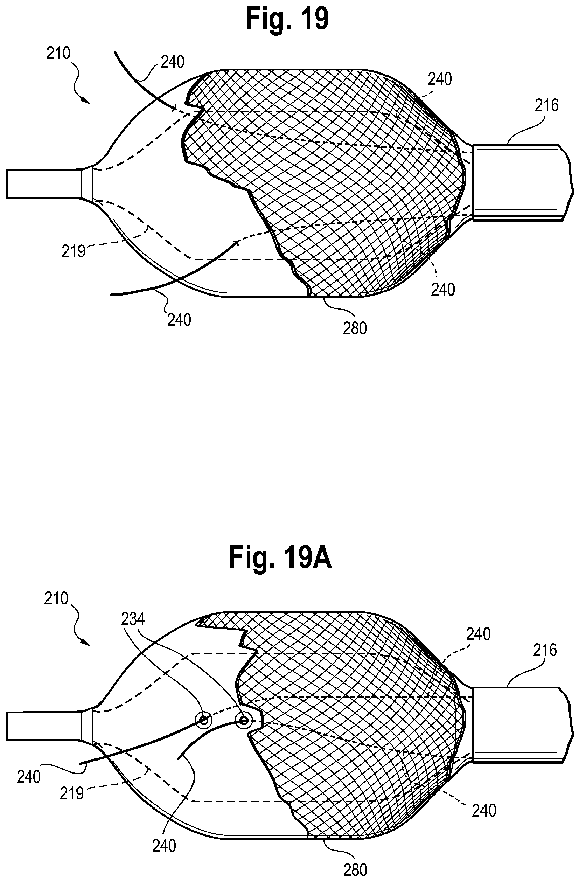

Similarly, with regard to the system 210, the mesh 270 could be replaced with a compliant balloon having mesh-like reinforced areas, rather than using a full mesh 270, which may be referred to as a mesh balloon 280. As shown in Figure 19, the mesh balloon 280 that surrounds the inner balloon 219 is generally completely covered by the mesh material, although Figure 19 illustrates a broken line to illustrate both the balloon material and the mesh material. This approach allows for puncturing holes at any desirable location in the mesh balloon 280. Figure 19A illustrates another embodiment where the mesh balloon 280 also includes predefined holes 234.

The mesh balloon 280 can be used in place of the mesh 270 described above and illustrated in the associated figures having the mesh 270. The function of the mesh 270 applies similarly to the mesh balloon 280. The mesh balloon 280 may be bonded to the outer shaft 272, similar to the mesh 270, and inflation fluid may be provided through the space between the shaft 216 and the outer shaft 272 to the mesh balloon 280. In another approach, the mesh balloon 280 may be bonded to the shaft 216, and the shaft 216 may include an additional inflation lumen in communication with the cavity 232 defined between the inner balloon 219 and the mesh balloon 280.

The mesh balloon 280 can include pre-formed holes 234 similar to those described above, or may be punctured at a reinforced area by the wires 240, and the mesh balloon 280 will provide the support for the daughter balloons 250, with the compliant inner balloon 219 providing the main inflation to expand the system into engagement with the vessel walls.

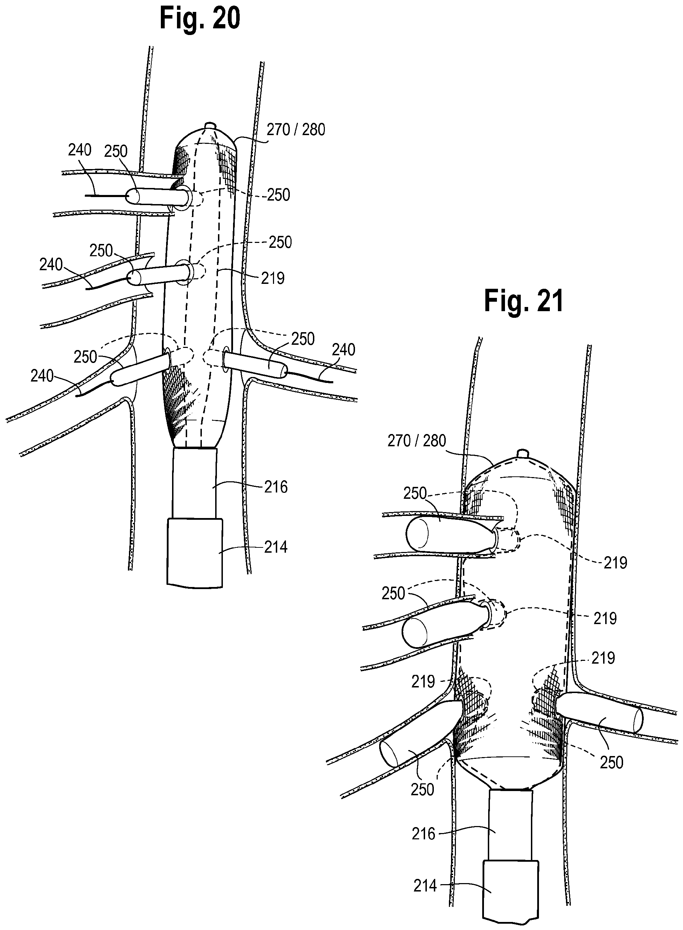

With reference to Figures 20 and 21, the system 210 can be delivered to the desired body vessel in a manner similar to that described above with relation to the system 10. For the sake of clarity, the mesh 270 or mesh balloon 280 is shown in a simplified form without illustrating the cells of the mesh.

The mesh 270 or mesh balloon 280 and the inner balloon 219 are delivered to the desired location where lumen support or dilation is desired. The wires 240, being already extend through the holes or disposed within the cavity of the mesh 270 or mesh balloon 280, are routed through the holes 234 and into the desired location, such as within a branch vessel. The daughter balloons 250 are delivered over the wires 240 and into the corresponding branch vessel, as well as extending through the holes 234. The daughter balloons 250 are inflated into engagement with the holes 234 such that the daughter balloons 250 are retained by the mesh 270 or mesh balloon 280 via the holes 234. The daughter balloons 250 may seal against the holes. However, in this embodiment, the daughter balloons 250 may not need to fully seal against the holes 234, as inflation and expansion is primarily provided by the inner balloon 219.

With the daughter balloons 250 retained by the mesh 270 or mesh balloon 280, as shown in Figure 20, the inner balloon 219 is then inflated and expanded into engagement with the body vessel wall, as shown in Figure 21. The inner balloon 219 does not include any holes, so inflation of the inner balloon 219 is not dependent on sufficient sealing between the daughter balloons 250 and their engagement with the holes 234 of the mesh 270 or mesh balloon 280.

With the balloon 219 being a compliant balloon, the balloon 219 will generally conform to the shape of the body vessel, as well as to the shape of the minimally compliant daughter balloons 250.

As described above, the delivery lumen 222 can be in the form of multiple dedicated delivery lumens 222a, b, c, and d. Each of these dedicated lumens can include a corresponding wire 240 that extends into the cavity 232 and optionally through a pre-defined hole 234. The daughter balloons 250 are deliverable through each of these dedicated lumens 222, allowing for delivery of multiple daughter balloons 250 at the same time. However, it will be appreciated that the balloons 250 could also be delivered sequentially through a single delivery lumen.

If additional inflation is desired beyond the inflation provided by the inner balloon 219, the mesh 270 or mesh balloon 280 may be inflated as well, depending on the sufficiency of the seal between the daughter balloons 250 and the holes 234 in the mesh 270 or mesh balloon 280. Thus, the inner balloon 219 provides the bulk of the inflation pressure, with the mesh 270 or mesh balloon 280 providing additional inflation if desired. Of course, in the case of a mesh 270, the openings through the cells of the mesh 270 may limit the effectiveness of inflation and may not be able to maintain a high inflation pressure, but the inflation that is performed within the mesh 270 may be sufficient to provide additional separation of movement of the mesh 270 in addition to the inflation pressure provided by the inner balloon 219.

The balloon 219 may be repeatedly inflated and deflated, while the daughter balloons 250 can remain inflated, due to the dedicated inflation lumens for each of the balloons.

Throughout this specification various indications have been given as to preferred and alternative examples and aspects of the invention. However, the foregoing detailed description is to be regarded as illustrative rather than limiting and the invention is not limited to any one of the provided aspects. It should be understood that it is the appended claims, including all equivalents, that are intended to define the scope of this invention.

The embodiments in United States patent application number US-15/631,788, form which this application claims priority, and in the abstract accompanying this application are incorporated herein by reference.

## Claims

1. A medical system comprising:
a balloon catheter including a shaft and a first inflatable balloon bonded to the shaft and a mesh bonded to the shaft and extending over and surrounding the first balloon;
a first inflation lumen extending within the shaft and in fluid communication with an interior cavity of the first balloon;
an intermediate space between an outer surface of the first balloon and an inner surface of the mesh;
at least one delivery lumen extending longitudinally through the shaft and in fluid communication with the intermediate space, the delivery lumen configured to deliver medical devices through the shaft and into the intermediate space; and
at least one daughter balloon moveable through the at least one delivery lumen and configured to extend through the mesh;
wherein the mesh retains the daughter balloon in position when the daughter balloon extends through the mesh and is inflated; and
wherein inflation of the first balloon expands the mesh.

2. A system according to claim 1, wherein the mesh includes at least one hole in a side of the mesh, wherein the hole is sized and configured to receive and retain the daughter balloon in position when the daughter balloon is inflated.

3. A system according to claim 1 or 2, wherein the shaft is a first shaft, the system comprising an outer shaft that surrounds the first shaft, and the mesh is bonded at one end to the first shaft and at an opposite end to the outer shaft.

4. A system according to claim 3, wherein the outer shaft and the first shaft have an annular space therebetween for delivering inflation fluid into the intermediate space.

5. A system according to any preceding claim, comprising at least one wire extending through the at least one delivery lumen and into the intermediate space, wherein the at least one daughter balloon includes a wire lumen and is deliverable over the wire into the intermediate space.

6. A system according to claim 5, wherein the wire extends through a side of the mesh.

7. A system according to any preceding claim, wherein the at least one delivery lumen comprises multiple delivery lumens, wherein the at least one daughter balloon comprises multiple daughter balloons each deliverable through one of the multiple delivery lumens.

8. A system according to any preceding claim, wherein the first balloon is compliant and the at least one daughter balloon is minimally compliant, wherein the first balloon conforms to the shape of the daughter balloon when inflated.

9. A medical system comprising:
a balloon catheter including a shaft and a first inflatable balloon bonded to the shaft and a mesh bonded to the shaft and extending over and surrounding the first balloon;
a first inflation lumen extending within the shaft and in fluid communication with an interior cavity of the first balloon;
an intermediate space between an outer surface of the first balloon and an inner surface of the mesh;
at least one delivery lumen extending longitudinally through the shaft and in fluid communication with the intermediate space, the delivery lumen configured to deliver medical devices through the shaft and into the intermediate space;
at least one daughter balloon moveable through the at least one delivery lumen and configured to extend through the mesh; and
at least one wire extending through the at least one delivery lumen and into the intermediate space;
wherein the system includes a delivery configuration with the first balloon and the mesh in a compressed delivery state and housed within a delivery sheath, and the system further includes a deployed configuration with the first balloon and the mesh exposed from the delivery sheath;
wherein, in the deployed configuration, the daughter balloon is deliverable over the at least one wire and extends through the mesh and is retained in position by the mesh when the daughter balloon is inflated; and
wherein, in the deployed configuration, inflation of the first balloon expands the mesh outward.

10. A system according to claim 9, wherein the at least one wire terminates within the intermediate space in the delivery configuration.

11. A system according to claim 9 or 10, wherein the wire is moveable through a side of the mesh in the deployed configuration.

12. A system according to claim 9, 10 or 11, wherein the wire extends through a side of the mesh in the delivery configuration.

13. A system according to claim 12, wherein a terminal end of the wire is attached to the shaft outside of the mesh at an insertion end of the shaft in the delivery configuration.

14. A system according to any preceding claim, wherein the mesh is coated.

15. A system according to any preceding claim, wherein the mesh is integrated with a second compliant balloon.
